# EUROPÄISCHE PATENTSCHRIFT

(11) **EP 1 255 401 B1**
(45) Veröffentlichungstag und Bekanntmachung des Hinweises auf die Patenterteilung: **27.07.2005**
(21) Anmeldenummer: 01110939.4
(22) Anmeldetag: 05.05.2001
(51) Int. Cl.: H04N 3/15, A61B 6/14

(54) **Gerät zur Bilderfassung im Oralbereich, insbesondere zur zahnmedizinischen Diagnose**
Device for recording images in the oral cavity, especially for dental diagnosis
Dispositif de captation d'images de la région buccale destiné, en particulier au diagnostic dentaire

(43) Veröffentlichungstag der Anmeldung: 06.11.2002
(73) Patentinhaber: Pfeiffer, Manfred, Dr., London W9 1EL (GB)
(72) Erfinder: Pfeiffer, Manfred, Dr., London W9 1EL (GB)
(74) Vertreter: Bahmann, Markus

(56) Entgegenhaltungen:
- EP-A- 0 714 632
- US-A- 5 677 537
- US-A- 5 691 539

## Beschreibung

Die Erfindung betrifft ein Gerät zur Bilderfassung im Oralbereich, insbesondere zur zahnmedizinischen Diagnose, mit einem Gehäuse, einem in dem Gehäuse angeordneten, flachen Bildsensor, dessen Sensorfläche als Rechteck mit schräg angeschnittenen Ecken gestaltet ist, wobei das Gehäuse in den den schräg geschnittenen Ecken entsprechenden Bereichen abgerundet geformt ist, einer Signalleitung, mittels der die Bildsignale des Bildsensors an eine getrennt angeordnete Bildverarbeitungs- und Speichereinheit übertragbar sind, wobei sich der Bildsensor aus matrixförmig angeordneten, optoelektronischen Halbleiterelementen sowie aus mindestens einem Ausleseregister zusammensetzt, welches die in den einzelnen Halbleiterelementen in Abhängigkeit von der jeweils empfangenen Strahlungsintensität gespeicherten Ladungen erfaßt.

Ein solches, auch als "Intraoralsensor" bezeichnetes Gerät ist z. B. aus der US-A 4,160,997 bekannt. Der Einsatz erfolgt beim Zahnarzt, um Röntgenaufnahmen von Zähnen oder kleineren Zahngruppen anzufertigen. Hierzu wird das Gehäuse des Sensors in den Mund des Patienten eingeführt und hinter dem zu durchleuchtenden Zahn oder der Zahngruppe positioniert, wobei der flach in dem Gehäuse angeordnete Bildsensor mit seiner strahlungsempfindlichen Schicht zu dem Zahn bzw. zu der Zahngruppe hin weist. Anschließend erfolgt von außerhalb die Beaufschlagung mit Röntgenstrahlen in geringer Dosis, wodurch die auf den Bildsensor auftreffenden optischen Impulse über eine aus dem Gehäuse herausgeführten Signalleitung zu einer Bildverarbeitungs- und Speichereinheit gelangen. Dies ist in der Regel ein PC, der über eine entsprechende Bildverarbeitungs-Software verfügt. Auf diese Weise können Röntgenbilder an Ort und Stelle sowie verzögerungsfrei aufgenommen und auf dem Bildschirm des Computers sichtbar gemacht werden. Der behandelnde Zahnarzt kann dann selbst entscheiden, welche ergänzenden Bilder eventuell noch erforderlich sind.

Nachteilig bei dem Gerät nach der US-A 4,160,997 ist die relativ eckige bzw. kantige Gestalt des Sensorgehäuses. Ursächlich hierfür ist die rechteckige Gestalt des verwendeten Bildsensors mit einem sich entlang der Kante der Sensorfläche erstreckenden Ausleseregister. Infolge der kantigen Form des Sensorgehäuses kann es beim Patienten zu Druckstellen und damit Schmerzen im Bereich der Mundhöhle oder des Zahnfleisches kommen.

In Bezug auf diese Nachteile ist in der EP 0 714 632 B1 ein weiterentwickeltes Gerät beschrieben, bei dem das Ausleseregister nicht entlang des Randes der Sensorfläche angeordnet ist, sondern in der Sensorfläche und vorzugsweise entlang dessen Mittelachse. Auf diese Weise ist es möglich, bei der Gestaltung der Sensorfläche von der Rechteckform abzuweichen und stattdessen die Sensorfläche mit schräg angeschnittenen Ecken zu versehen, wobei sich das Gehäuse in den den schräg geschnittenen Ecken entsprechenden Bereichen abgerundet formen läßt. Nachteilig bei diesem Gerät ist der sich entlang der Achse im Bereich des zentralen Ausleseregisters zwangsläufig ausbildende Lesefehler, da entlang dieser Achse Bildsignale nicht erfaßt werden können. In der Praxis wird daher bei dem bekannten Gerät der Weg beschritten, auf programmtechnischem Wege diese Defizite auszugleichen, etwa durch Interpolation oder Bildung geeigneter Mittelwerte. Damit verbunden ist ein hoher programmtechnischer Aufwand innerhalb der Bildverarbeitungs-Software.

Der Erfindung liegt die **Aufgabe** zugrunde, einen Intraoralsensor zu schaffen, der eine korrektions- bzw. interpolationsfreie Verarbeitung der Signale der Bildpunkte ermöglicht, und sich durch ein für den Patienten angenehmes Tragegefühl auszeichnet.

Zur **Lösung** dieser Aufgabe wird bei einem Gerät zur Bilderfassung der eingangs genannten Art vorgeschlagen, daß das Ausleseregister entlang eines Außenrandes der Sensorfläche angeordnet und mit mindestens einer Abknickung versehen ist, wobei sich der eine Arm der Abknickung entlang eines der schräg angeschnittenen Ränder der Sensorfläche erstreckt.

Das erfindungsgemäße Gerät verwendet daher einen Bildsensor, bei dem sich das Ausleseregister entlang des Randes der Sensorfläche erstreckt. Im Gegensatz zu Intraoralsensoren mit mittig angeordneten Ausleseregistern ist auf diese Weise eine korrektions- bzw. interpolationsfreie Verarbeitung der Signale der von den Bildpunkten (Pixel) erzeugten Signale möglich. Obwohl sich das Ausleseregister entlang des Randes der Sensorfläche erstreckt, wird eine Beeinträchtigung des Tragekomforts durch rechteckige Übergänge zwischen den Rändern der Sensorfläche vermieden, da erfindungsgemäß die Sensorfläche als Rechteck mit schräg angeschnittenen Ecken gestaltet ist, und das Gehäuse des Intraoralsensors an den den schräg geschnittenen Ecken entsprechenden Stellen abgerundet geformt ist. Zur Realisierung dieser Randgestaltung der Sensorfläche ist das Ausleseregister mit mindestens einer Abknickung versehen, und zumindest ein Arm der Abknickung erstreckt sich entlang eines der schräg angeschnittenen Ränder der Sensorfläche. Der Winkel des schräg angeschnittenen Randes gegenüber der exakten Rechteckform beträgt vorzugsweise 45°, jedoch lassen sich auch mit Winkeln von 30° bis 60° Übergänge zwischen den Haupträndern der Sensorfläche erzeugen, die eine abgerundete Gestaltung des Gehäuses des Intraoralsensors gestatten. Entsprechend beträgt auch jede Abknickung des Ausleseregisters entlang des Außenrandes der Sensorfläche vorzugsweise 45°, in jedem Fall aber zwischen 30° und 60°.

Da das Gehäuse mit relativ großzügigen Rundungen versehen werden kann, erzeugt dieses bei der Anwendung im Zahnbereich des Patienten weniger Druckstellen als die bekannten, aufgrund der Verwendung eines rechteckigen Sensors wesentlich kantiger gestalteten Sensorgehäuses, so daß sich der Tragekomfort insgesamt verbessert.

Gemäß einer bevorzugten Ausgestaltung des erfindungsgemäßen Gerätes erstreckt sich das Ausleseregister sowohl entlang eines mittleren Randabschnittes der Sensorfläche, der mit der rechteckigen Grundform der Sensorfläche zusammenfällt, als auch entlang der sich beiderseits über Abknickungen an den mittleren Randabschnitt anschließenden Ränder der schräg angeschnittenen Ecken. Auf diese Weise ist es möglich, sämtliche Pixel der Sensorfläche mit einem einzigen Ausleseregister auszulesen, wobei dieses Ausleseregister mit mindestens zwei Abknickungen versehen ist.

Weitere Einzelheiten eines erfindungsgemäß ausgebildeten Intraoralsensors werden nachfolgend anhand der zugehörigen Zeichnung erläutert. Auf der Zeichnung zeigen:
- Fig. 1: in einer Ansicht einen Intraoralsensor mit einer aus Übersichtsgründen geschnittenen Zuleitung;
- Fig. 2: einen Schnitt entlang der Linie II-II der Fig. 1;
- Fig. 3: in um 180° gedrehter Ansicht einen in dem Gerät nach den Fign. 1 und 2 verwendeten Bildsensor;
- Fig. 4: einen sehr stark vergrößerten Ausschnitt aus dem Bildsensor nach Fig 3, wobei dieser Ausschnitt in Fig. 3 mit IV bezeichnet ist;
- Fig. 5: eine gegenüber Fig. 3 geänderte Ausführungsform des Bildsensors und
- Fig. 6: eine nochmals geänderte Ausführungsform des Bildsensors.

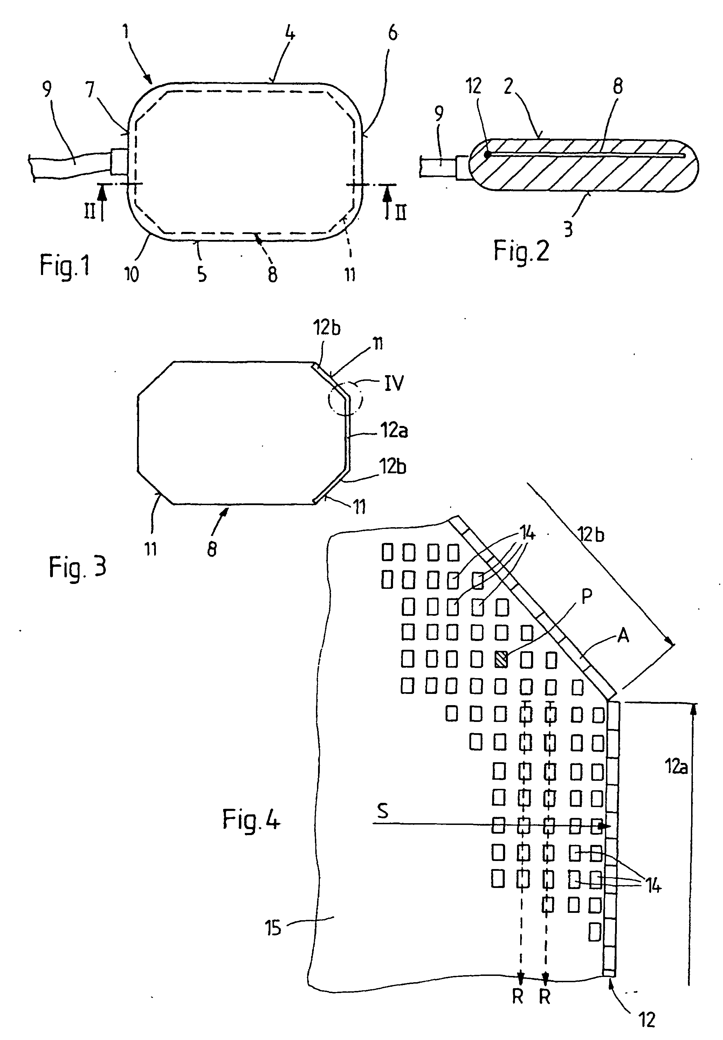

Das in den Fign. 1 und 2 dargestellte Gerät besteht aus einem im wesentlichen rechteckigen Gehäuse 1 mit Grundabmessungen von etwa 25 mm x 32 mm x 7,5 mm. Das Gehäuse 1 verfügt über zwei Hauptseiten 2, 3, zwei längere Schmalseiten 4, 5 und zwei kürzere Schmalseiten 6, 7. Nahe der einen Hauptseite 2 ist in dem Gehäuse 1 ein auf Röntgenstrahlen sensibilisierter Bildsensor 8 angeordnet. Mittels einer Signalleitung in Gestalt eines flexiblen Kerbels 9 lassen sich die Signale des Bildsensors 8 einer auf der Zeichnung nicht dargestellten Bildverarbeitungs- und Speichereinheit zuführen. Ebenfalls auf der Zeichnung nicht dargestellt ist die in Richtung auf den Bildsensor 8 ausgerichtete Röntgenstrahlenquelle. Bei der Anwendung wird das Gerät mit dem nach vorne gerichteten Bildsensor so in den Mund des Patienten eingesetzt, daß der zu durchleuchtende Zahnkieferbereich zwischen Röntgenquelle und Bildsensor 8 liegt. Die genaue Ausrichtung der Achsen der Röntgenstrahlenquelle einerseits und des Bildsensors 8 andererseits kann mittels bekannter Zentriereinrichtungen erfolgen.

Sämtliche Ecken des die Grundform eines Rechtecks aufweisenden Gehäuses 1 sind mit großzügigen Rundungen 10 versehen. Diese Rundungen sind möglich, da der flach in dem Gehäuse angeordnete Bildsensor 8 zwar ebenfalls die Grundgestalt eines Rechtecks hat, jedoch die Ränder dieses Rechtecks im Bereich der Ecken 11 innerhalb der Rundungen 10 des Gehäuses schräg angeschnitten sind, vorzugsweise unter einem Winkel von 45°.

Das Ausleseregister 12 des Bildsensors 8 ist, wie Fig. 2 erkennen läßt, entlang eines Teils des Randes des Bildsensors angeordnet. Die Funktion des Bildsensors 8 sowie die Bedeutung des Ausleseregisters 12 wird nachfolgend anhand der Fig. 4 erläutert. Einzelheiten zur technischen Durchführung finden sich z. B. in dem Fachbuch von Phillip E. Mattison "Practical digital video with programming examples in C", erschienen im Verlag John Wiley & Sons, Inc.

Der im Ausführungsbeispiel dargestellte Bildsensor ist vom sogenannten "CCD"-Typ (Charge Coupled Device). Die achteckige Sensorfläche ist mit Bildpunkten ("Pixels") versehen, die in Reihen R und Spalten S angeordnet sind, so daß sich insgesamt die Struktur einer Matrix ergibt. Jeder Bildpunkt besteht aus einem diskreten Halbleiterelement 14, welches sich aus einem strahlungsempfindlichen Element sowie einem Speicherelement zusammensetzt. Sämtliche Halbleiterelemente 14 sind auf einem gemeinsamen Substrat 15 aus vorzugsweise Silikon angeordnet.

Das in den Fign. 3 und 4 dargestellte Ausleseregister 12 ist geknickt und setzt sich aus einem Mittelabschnitt 12a und an beiden Enden des Mittelabschnitts 12a sich anschließenden Seitenarmen 12b zusammen. Mittelabschnitt 12a und Seitenarme 12b folgen den jeweiligen Rändern des Bildsensors 8, wodurch sich ein insgesamt abgeknicktes Ausleseregister 12 mit einem Knick zwischen dem Mittelabschnitt 12a und dem jeweiligen Seitenarm 12b ergibt. Auch der Winkel dieser Abknickung beträgt wiederum 45°, zumindest jedoch zwischen 30° und 60°.

Fig. 4 läßt erkennen, daß die Abknickung der Ränder entlang der Seitenarme 12b keinen Einfluß auf die Ausrichtung der Pixelreihen R hat. Bei der dargestellten Ausführungsform mit im wesentlichen quadratischen Pixels enthält allerdings jede Spalte S der Seitenarme 12b, von dem Mittelabschnitt 12a aus gesehen, einen Pixel weniger, als die jeweils vorangehende Spalte. Berücksichtigt man noch die entsprechende Gestaltung der Eckenränder am anderen Ende der Spalten, ergibt sich sogar von Spalte zu Spalte eine Reduzierung der Pixelzahl um zwei. Bei der rechnerischen Auswertung der Bildpunkt-Signale des Bildsensors sind diese geometrischen Verhältnisse in geeigneter Weise zu berücksichtigen. So ist z. B. das in Fig. 4 beispielsweise gekennzeichnete Pixel P in der fünften Reihe R, bezogen auf den Mittelabschnitt 12a des Ausleseregisters angeordnet, jedoch nur in dritter Position bezogen auf jene Spalte, in der sich dieses Pixel P befindet.

Die auf die einzelnen Halbleiterelemente 14 bzw. Pixel auftreffenden Röntgenstrahlen führen dazu, daß in dem strahlungsempfindlichen Element des jeweiligen Halbleiterelements Ladungsteilchen frei werden, die in das zugehörige Speicherelement gelangen. Wird das betreffende Halbleiterelement 14 abgefragt, gelangen die so vorläufig gespeicherten Ladungsimpulse in das jeweilige Register, im Fall des Pixels P in das Register A, um dann als Bildsignal weiterverarbeitet zu werden. Die einzelnen Halbleiterelemente 14 werden Reihe R für Reihe R durch das jeweilige Einzelregister des Ausleseregisters 12 abgefragt, so daß die Leserichtung gleich ist der Ausrichtung der Spalten S.

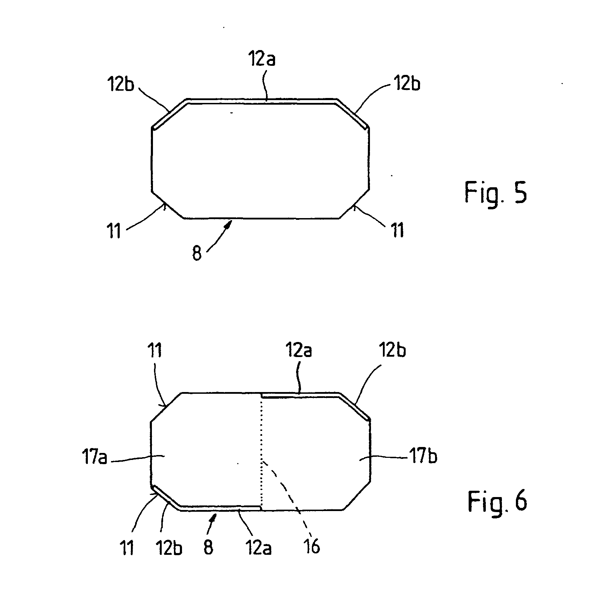

In Fig. 5 ist anhand einer geänderten Ausführungsform dargestellt, daß sich das aus Mittelabschnitt 12a sowie den Seitenarmen 12b zusammensetzende Ausleseregister 12 auch entlang der längeren Seite des Rechtecks erstrecken kann.

In Fig. 6 schließlich ist eine Ausführungsform dargestellt, bei der die Sensorfläche des Bildsensors in zwei durch die Mittellinie 16 getrennte Teilflächen 17a, 17b aufgeteilt ist. Die Pixel in Teilfläche 17a werden von dem am unteren Rand angeordneten Ausleseregister mit Abschnitt 12a und Seitenarm 12b ausgelesen, die Pixel in der anderen Teilfläche 17b von dem Abschnitt 12a sowie dem angrenzenden Seitenarm 12b am oberen Rand. Hier werden also zwei getrennte Ausleseregister verwendet, die sich entlang einander gegenüberliegender Hauptränder der Sensorfläche erstrecken.

### Bezugszeichenliste

- 1: Gehäuse
- 2: Hauptseite
- 3: Hauptseite
- 4: Schmalseite
- 5: Schmalseite
- 6: Schmalseite
- 7: Schmalseite
- 8: Bildsensor
- 9: Kabel
- 10: Rundung
- 11: Ecke, schräg angeschnitten
- 12: Ausleseregister
- 12a: Mittelabschnitt des Ausleseregisters
- 12b: Seitenarm des Ausleseregisters
- 14: Halbleiterelement, Pixel
- 15: Substrat
- 16: Mittellinie
- 17a: Teilfläche
- 17b: Teilfläche

- A: Einzelregister des Ausleseregisters
- P: einzelnes Pixel
- R: Reihe mit Halbleiterelementen
- S: Spalte mit Halbleiterelementen

## Patentansprüche

1. Gerät zur Bilderfassung im Oralbereich, insbesondere zur zahnmedizinischen Diagnose, mit einem Gehäuse (1), einem in dem Gehäuse (1) angeordneten, flachen Bildsensor (8), dessen Sensorfläche als Rechteck mit schräg angeschnittenen Ecken (11) gestaltet ist, wobei das Gehäuse (1) in den den schräg angeschnittenen Ecken entsprechenden Bereichen abgerundet geformt ist, einer Signalleitung, mittels der die Bildsignale des Bildsensors (8) an eine getrennt angeordnete Bildverarbeitungs- und Speichereinheit übertragbar sind, wobei sich der Bildsensor (8) aus matrixförmig angeordneten, optoelektronischen Halbleiterelementen (14) sowie mindestens einem Ausleseregister (12) zusammensetzt, welches die in den einzelnen Halbleiterelementen (14) in Abhängigkeit von der jeweils empfangenen Strahlungsintensität gespeicherten Ladungen erfaßt,
**dadurch gekennzeichnet,**
**daß** das Ausleseregister (12) entlang eines Außenrandes der Sensorfläche angeordnet und mit mindestens einer Abknickung versehen ist, wobei sich der eine Arm (12b) der Abknickung entlang eines der schräg angeschnittenen Ränder (11) der Sensorfläche erstreckt.

2. Gerät zur Bilderfassung nach Anspruch 1, **dadurch gekennzeichnet, daß** sich das Ausleseregister (12) sowohl entlang eines mittleren Randabschnitts (12a) der Sensorfläche, der mit der rechteckigen Grundform der Sensorfläche zusammenfällt, als auch entlang der sich beiderseits über Abknickungen an den mittleren Randabschnitt (12a) anschließenden Ränder der schräg angeschnittenen Ecken (11) erstreckt.

## Revendications

1. Dispositifs destinés à l'enregistrement d'image dans la région orale, plus particulièrement à des diagnostics médico-dentaires, composés d'une enveloppe (1), d'un capteur d'image (8) plat intégré à l'enveloppe (1) et dont la surface est en forme de rectangle aux angles (11) coupés en biais, laquelle enveloppe (1) est arrondie au niveau des zones correspondant aux angles (11) coupés en biais, d'une ligne d'acheminement des signaux au moyen duquel les signaux d'images émis par le capteur d'image (8) sont transmis à une unité de traitement d'images et de mémorisation séparée, le capteur d'image (8) étant lui-même composé d'éléments semi-conducteurs (14) optoélectroniques formant une matrice et d'au moins un dispositif de lecture (12), lequel répertorie les charges enregistrées dans les éléments semi-conducteurs (14) isolés en fonction de l'intensité de rayonnement reçue,
**caractérisés,**
**en ce que** le système de lecture (12) est disposé sur l'un des bords extérieurs de la surface du capteur et comporte au moins un pli, un retour (12b) du pli s'étendant sur l'un des bords (11) coupés en biais de la surface du capteur.

2. Dispositif destiné à l'enregistrement d'image selon la revendication 1, **caractérisé en ce que** le système de lecture (12) s'étend non seulement sur une partie centrale du bord (12a) de la surface du capteur, correspondant à la forme rectangulaire de base de la surface du capteur, mais également sur les bords des angles (11) coupés en biais et contigus à la partie centrale du bord (12a) au-delà des deux plis.

## Claims

1. Devices for capture of images in the oral region, especially for dental diagnosis, with a housing (1), a flat image sensor (8) arranged in the housing (1), the capturing area of rectangular design with chamfered corners (11), the housing (1) being rounded in the areas corresponding to the chamfered corners, a signal line by means of which the image signals from the image sensor (8) can be transferred to an image processing and memory unit arranged separately, the image sensor (8) being composed of opto-electronic semi-conductor elements (14) in matrix arrangement, as well as at least one readout register (12) which detects the charges stored in the individual semi-conductor elements (14) as a function of the radiation intensity received,
**characterized in that**
the readout register (12) is arranged along one of the outer edges of the capturing area and features at least one kink, with the one arm (12b) of the kink reaching along one of the chamfered edges (11) of the capturing area.

2. Device for capturing images according to Claim 1, **characterized in that** the readout register (12) stretches along the middle section of the edge (12a) of the capturing area, which coincides with the basic rectangular shape of the sensor surface, as well as along the edges of the chamfered corners (11), the edges being contiguous to the central part of the edge on both sides.
